# EUROPEAN PATENT APPLICATION

(11) **EP 3 260 045 A1**
(43) Date of publication of application: **27.12.2017**
(21) Application number: 16890124.7
(22) Date of filing: 22.03.2016
(51) Int. Cl.: A61B 5/113, A61B 5/08

(54) **APNEA DETECTION DEVICE**

(71) Applicant: E3 Co., Ltd., Tokyo 163-0532 (JP)
(72) Inventor: YAO, Bingwei, Tokyo 163-0532 (JP)
(74) Representative: Wittmann, Günther
(86) International application number: PCT/JP2016/058926
(87) International publication number: WO 2017/163301

(57) **Abstract**

There is provided a detecting unit 13 for detecting an apnea condition of a user based on correlation of acceleration information to be measured by an acceleration sensor 11 for detecting a motion of a body as an acceleration and angular velocity information to be measured by an angular velocity sensor 12 for detecting the motion of the body as an angular velocity. By deciding that the apnea condition is brought only when both an acceleration to be measured by the acceleration sensor 11 and an angular velocity to be measured by the angular velocity sensor 12 indicate the apnea condition, it is possible to detect apnea during sleeping more accurately.

## Description

### TECHNICAL FIELD

The present invention relates to an apnea detecting apparatus for detecting an apnea condition in attachment to a body of a user for a sleeping period.

### BACKGROUND ART

A sleep apnea syndrome is a disease in which respiratory arrest or hypopnea is caused during sleeping. This disease has weak subjective symptoms in many cases. In contrast, conventionally, there is known the technique in which a body motion or an acceleration during sleeping is detected to detect an apnea condition (for example, see Patent Documents 1 to 3).

The sleep apnea testing apparatuses described in the Patent Documents 1 to 3 include a three-dimensional acceleration sensor and signal processing means for detecting a sleeping posture of a patient from a DC component obtained from the acceleration sensor and detecting a respiration rate or a heart rate of a patient from an AC component obtained from the acceleration sensor and can acquire necessary information for a sleep test or a sleep apnea test from a single acceleration sensor.

Patent Document 1 : Japanese Laid-Open Patent Publication No. 2006 - 247374
Patent Document 2 : Japanese Laid-Open Patent Publication No. 2006 - 320733
Patent Document 3 : Japanese Laid-Open Patent Publication No. 2006 - 320734

### DISCLOSURE OF THE INVENTION

Referring to the Patent Documents 1 to 3, body motion data based on respiration to be calculated by performing a predetermined calculation over the AC component to be obtained from the acceleration sensor is compared with body motion data during respiration and body motion data during apnea stored in a database to estimate either a respiratory condition or an apnea condition.

However, an acceleration to be obtained by respiration does not have a very large value. The deciding method using simple data comparison described above has a problem in that either the respiratory condition or the apnea condition is estimated by mistake.

The present invention has been made to solve the problem and has an object to enable more accurate detection of apnea during sleeping.

In order to attain the object, the present invention serves to detect an apnea condition of a user based on correlation of acceleration information to be measured by an acceleration sensor for detecting a motion of a body as an acceleration and angular velocity information to be measured by an angular velocity sensor for detecting a motion of a body as an angular velocity.

According to the present invention having the structure described above, it is decided that the apnea condition is brought only when both the acceleration to be measured by the acceleration sensor and the angular velocity to be measured by the angular velocity sensor indicate the apnea condition. Therefore, it is possible to detect apnea during sleeping more accurately.

### BRIEF DESCRIPTION OF THE DRAWINGS

FIG. 1 is a block diagram showing an example of a functional structure of an apnea detecting apparatus according to the present embodiment.
FIG. 2 is a view showing an example of an appearance structure of the apnea detecting apparatus according to the present embodiment.

### BEST MODE FOR CARRYING OUT THE INVENTION

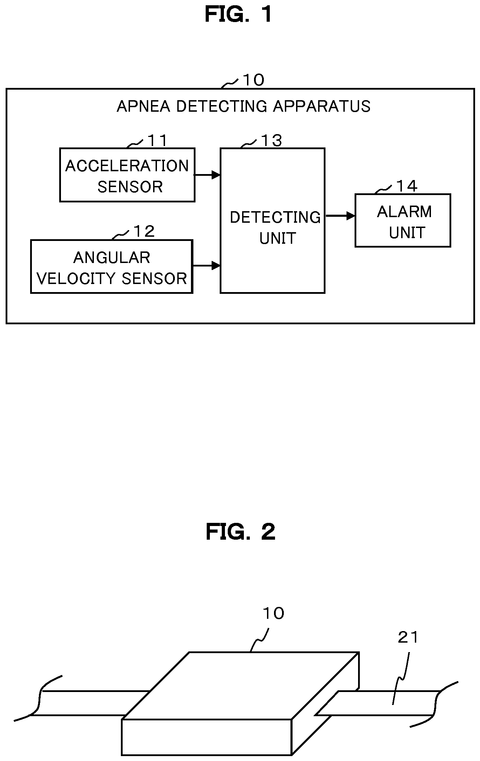

An embodiment of the present invention will be described below with reference to the drawings. FIG. 1 is a block diagram showing an example of a functional structure of an apnea detecting apparatus according to the present embodiment. FIG. 2 is a view showing an example of an appearance structure of the apnea detecting apparatus according to the present embodiment.

An apnea detecting apparatus 10 according to the present embodiment serves to detect an apnea condition in attachment to a body of a user for a sleeping period. As shown in FIG. 2, the apnea detecting apparatus 10 according to the present embodiment is formed to be portable and is attached to a chest portion or an abdomen portion of a user to detect an apnea condition of the user, for example.

There is provided a belt 21 for fixing the apnea detecting apparatus 10 to a body in such a manner that the apnea detecting apparatus 10 does not slip off the body during sleeping. Although the belt 21 is used, a fixing method is not restricted thereto. For example, the apnea detecting apparatus 10 may be fixed to the body by using a tape or the like.

As shown in FIG. 1, the apnea detecting apparatus 10 according to the present embodiment includes an acceleration sensor 11, an angular velocity sensor 12, a detecting unit 13 and an alarm unit 14 as a functional structure thereof. The detecting unit 13 and the alarm unit 14 can also have a part or all of functions constituted by any of hardware, DSP (Digital Signal Processor) and software.

In the case in which they are constituted by software, for example, each of the function blocks 13 and 14 actually includes a CPU, an RAM, an ROM and the like in a computer and is implemented by an operation of a program stored in a storage medium such as an RAM, an ROM, a hard disk or a semiconductor memory.

The acceleration sensor 11 detects a motion of a body of a user as an acceleration. With a transverse direction (a body width direction) of the user set to be an X axis, a longitudinal direction (a height direction) of the user set to be a Y axis and a vertical direction (a body thickness direction connecting an abdomen portion and a back portion) of the user set to be a Z axis, the acceleration sensor 11 to be used may be of any type such as 1-axis to 3-axis. However, at least an acceleration in a Z-axis direction (a body thickness direction) is to be detected. The reason is that the apnea detecting apparatus 10 fixed to the chest portion or abdomen portion is moved vertically in the body thickness direction when the user breathes.

The angular velocity sensor 12 detects the motion of the body of the user as an angular velocity. Although the angular velocity sensor 12 to be used may be of any type such as 1-axis to 3-axis, at least an angular velocity with the X axis set to be a rotation axis (an angular velocity of a rotation angle on a Y - Z plane) is to be detected. The reason is that the apnea detecting apparatus 10 fixed to the chest portion or the abdomen portion is vertically moved in the body thickness direction with inclination to the longitudinal direction when the user breathes.

The detection of the acceleration through the acceleration sensor 11 and the detection of the angular velocity through the angular velocity sensor 12 are continuously performed during the sleep of the user.

The detecting unit 13 detects the apnea condition of the user based on the correlation of the acceleration information to be measured by the acceleration sensor 11 and the angular velocity information to be measured by the angular velocity sensor 12. Specifically, the detecting unit 13 detects, as the apnea condition, a period in which a value of the acceleration is equal to or smaller than a threshold and that of the angular velocity is equal to or smaller than a threshold. In the case in which the acceleration sensor 11 or the angular velocity sensor 12 detects values in plural axis directions, the detecting unit 13 decides that the apnea condition is brought when all of the values in the plural axis directions are equal to or smaller than a threshold.

The alarm unit 14 outputs an alarm when the apnea condition is detected by the detecting unit 13. The alarm is made by causing a vibrator to perform vibration, for example. Alternatively, a predetermined alarm sound may be output from a speaker.

As described above in detail, the apnea detecting apparatus 10 according to the present embodiment serves to detect the apnea condition of the user based on the correlation of the acceleration information to be measured by the acceleration sensor 11 for detecting the motion of a body as an acceleration and the angular velocity information to be measured by the angular velocity sensor 12 for detecting the motion of the body as an angular velocity.

According to the apnea detecting apparatus 10 in accordance with the present embodiment having such a structure, only when both the acceleration to be measured by the acceleration sensor 11 and the angular velocity to be measured by the angular velocity sensor 12 indicate the apnea condition, it is decided that the apnea condition is brought. Therefore, the apnea during sleeping can be detected more accurately.

The body motion is made feeble depending on a sleeping way of the user so that the value of the acceleration to be detected by the acceleration sensor 11 is reduced. Also in that case, the angular velocity is also detected by the angular velocity sensor 12 in the present embodiment. Even if the value to be detected is small, therefore, the apnea can be detected based on the correlation between both of them.

Although the description has been given to the example in which the alarm unit 14 is provided in the embodiment, the present invention is not restricted thereto. For example, an information recording unit may be provided in place of the alarm unit 14 to record, in a storage medium, the acceleration information to be measured by the acceleration sensor 11 and the angular velocity information to be measured by the angular velocity sensor 12 during sleeping. Consequently, the recorded information can be output to an external computer which is not shown and be thus utilized. Alternatively, the apnea detecting apparatus 10 itself may be provided with a display to enable graphical visualization and display of the information recorded in the storage medium.

In addition, each of the embodiment is only illustrative for concreteness to carry out the present invention and the technical scope of the present invention should not be thereby construed to be restrictive. In other words, the present invention can be carried out in various configurations without departing from the gist or main features thereof.

### EXPLANATION OF DESIGNATION

- 10: apnea detecting apparatus
- 11: acceleration sensor
- 12: angular velocity sensor
- 13: detecting unit
- 14: alarm unit

## Claims

1. An apnea detecting apparatus for detecting an apnea condition in attachment to a body of a user for a sleeping period, the apparatus comprising:
an acceleration sensor for detecting a motion of the body as an acceleration;
an angular velocity sensor for detecting the motion of the body as an angular velocity; and
a detecting unit for detecting an apnea condition of the user based on correlation of acceleration information to be measured by the acceleration sensor and angular velocity information to be measured by the angular velocity sensor.

2. The apnea detecting apparatus according to claim 1, wherein the detecting unit detects, as the apnea condition, a period in which a value of the acceleration is equal to or smaller than a threshold and a value of the angular velocity is equal to or smaller than a threshold.

3. The apnea detecting apparatus according to claim 1, wherein an acceleration in a body thickness direction of the user is detected by the acceleration sensor and an angular velocity with a body width direction of the user set to be a rotation axis is detected by the angular velocity sensor.

4. The apnea detecting apparatus according to claim 1 further comprising an alarm output unit for outputting an alarm when the apnea condition is detected by the detecting unit.
